# EUROPÄISCHE PATENTSCHRIFT

(11) **EP 2 300 621 B1**
(45) Veröffentlichungstag und Bekanntmachung des Hinweises auf die Patenterteilung: **26.02.2014**
(21) Anmeldenummer: 09780678.0
(22) Anmeldetag: 15.07.2009
(51) Int. Cl.: C12Q 1/68, C12N 5/00

(54) **VERFAHREN ZUR ZELLIDENTIFIKATION UND ZELLSORTIERUNG**
METHOD FOR CELL IDENTIFICATION AND CELL SORTING
PROCÉDÉ D'IDENTIFICATION ET DE TRI DE CELLULES

(30) Priorität: 15.07.2008 DE 102008033070; 17.07.2008 DE 102008033570
(43) Veröffentlichungstag der Anmeldung: 30.03.2011
(73) Patentinhaber: Masterrind GmbH, 27283 Verden (DE)
(72) Erfinder: RATH, Detlef, 31535 Neustadt (DE); KUES, Wilfried, 31535 Neustadt (DE); TAYLOR, Ulrike, 31535 Neustadt (DE); BARCIKOWSKI, Stephan, 30419 Hannover (DE); PETERSEN, Svea, 18057 Rostock (DE)
(74) Vertreter: Taruttis, Stefan Georg
(86) Internationale Anmeldenummer: PCT/EP2009/059117
(87) Internationale Veröffentlichungsnummer: WO 2010/007118

(56) Entgegenhaltungen:
- EP-A1- 0 235 046
- US-A- 5 840 504
- DE LARA JOCELYN ET AL: "Fluorescent in situ hybridization of the telomere repeat sequence in hamster sperm nuclear structures" JOURNAL OF CELLULAR BIOCHEMISTRY, Bd. 53, Nr. 3, 1993, Seiten 213-221, XP009125094 ISSN: 0730-2312
- GARNER ET AL: "Flow cytometric sexing of mammalian sperm" THERIOGENOLOGY, LOS ALTOS, CA, US, Bd. 65, Nr. 5, 15. März 2006 (2006-03-15), Seiten 943-957, XP005297930 ISSN: 0093-691X
- "ClustalW2" INTERNET CITATION 29. Oktober 2004 (2004-10-29), Seiten 1-3, XP007910362 Gefunden im Internet: URL:http://www.ebi.ac.uk/Tools/es/cgi-bin/ clustalw2/result.cgi?tool=cl> [gefunden am 2009-10-29]
- PERRET J ET AL: "A polymorphic satellite sequence maps to the pericentric region of the bovine Y chromosome" GENOMICS, ACADEMIC PRESS, SAN DIEGO, US, Bd. 6, Nr. 3, 1. März 1990 (1990-03-01), Seiten 482-490, XP024797484 ISSN: 0888-7543 [gefunden am 1990-03-01]
- "Bovine Y-chromosome specific DNA probe, SEQ ID 1" INTERNET CITATION 14. Februar 2006 (2006-02-14), Seite 1, XP007910363 Gefunden im Internet: URL:http://www.ebi.ac.uk/cgi-bin/epo/epofe tch?id=AFG24798> [gefunden am 2009-10-30]
- JOHNSON L A ET AL: "Preselection of sex of offspring in swine for production: current status of the process and its application" THERIOGENOLOGY, LOS ALTOS, CA, US, Bd. 63, Nr. 2, 15. Januar 2005 (2005-01-15), Seiten 615-624, XP025259547 ISSN: 0093-691X [gefunden am 2005-01-15]
- HABERMANN F A ET AL: "Validation of sperm sexing in the cattle (Bos taurus) by dual colour fluorescence in situ hybridization." JOURNAL OF ANIMAL BREEDING AND GENETICS = ZEITSCHRIFT FÜR TIERZÜCHTUNG UND ZÜCHTUNGSBIOLOGIE APR 2005, Bd. 122 Suppl 1, April 2005 (2005-04), Seiten 22-27, XP009125095 ISSN: 0931-2668
- Jasmine I. Daksis ET AL: "Heteropolymeric Triplex-Based Genomic Assay to Detect Pathogens or Single-Nucleotide Polymorphisms in Human Genomic Samples", PLoS ONE, vol. 2, no. 3, 21 March 2007 (2007-03-21), page e305, XP55037257, DOI: 10.1371/journal.pone.0000305
- FRANK-KAMENETSKII M D ET AL: "TRIPLEX DNA STRUCTURES", ANNUAL REVIEW OF BIOCHEMISTRY, PALTO ALTO, CA, US, vol. 64, 1 January 1995 (1995-01-01), pages 65-95, XP001122079, ISSN: 0066-4154, DOI: 10.1146/ANNUREV.BI.64.070195.000433
- GARY D BURKHOLDER ET AL: "Immunofluorescent localization of triplex DNA in polytene chromosomes of Chironomus and Drosophila", CHROMOSOMA, SPRINGER, DE, vol. 101, no. 1, 1 October 1991 (1991-10-01), pages 11-18, XP009162506, ISSN: 0009-5915

## Beschreibung

Die vorliegende Erfindung betrifft ein Verfahren und in dem Verfahren verwendbare Verbindungen zur Analyse von Zellen aufdas Vorliegen eines Analyten. Die in dem Verfahren verwendeten Verbindungen zum Nachweis eines Analyten sind optisch detektierbar und enthalten einen Bindeanteil, der spezifisch einen Analyten binden kann. Bevorzugt ist der Bindeanteil ein Oligonukleotid, auch als Nukleinsäuresequenz bezeichnet.

Die Verwendung der Detektionskonjugate in dem erfindungsgemäßen Verfahren erlaubt die Detektion von Analyten und insbesondere die Identifikation und Sortierung bzw. Selektion von Zellen anhand eines spezifischen Analyten durch eine in dem Detektionskonjugat enthaltene detektierbare Markierung. Entsprechend betrifft die Erfindung auch die durchflusszytometrische Analyse von Zellen, optional mit anschließender Fraktionierung der Zellen in Abhängigkeit von dem detektierten Signal, z.B. durch Ablenkung der Zellen in separate Fraktionen oder durch thermische Behandlung von Zellen in Abhängigkeit von dem zellspezifisch detektierten Signal, wobei die Zellen vorzugsweise zur Detektion und zur anschließenden Fraktionierung bzw. Behandlung durch Beabstandung in einzelnen Tropfen oder in einer kontinuierlichen Transportflüssigkeit räumlich getrennt sind.

### Stand der Technik

Die WO 2008/060713 beschreibt die Sortierung von Zellen mittels Durchflusszytometrie, wobei Zellen mit Nanopartikeln markiert sind, die für einen Zelltyp spezifische Bindemoleküle tragen. Vorzugsweise sind die Nanopartikel aus Gold. Die Detektion gebundener Nanopartikel erfolgt durch Bestimmung der Masse, vorzugsweise wird die Masse als Änderung der Resonanzfrequenz bestimmt, wenn der spezifische Zelltyp an eine spezifische funktionalisierte Wandung eines Resonators bindet.

Die WO 2006/012597 beschreibt Verfahren zur Anreicherung X- oder Y-Chromosomen-haltiger Spermien unter anderem durch optische Identifizierung der Position einer Spermaspezies in einer Dispersion nach spezifischer Markierung und Erwärmen der Positionen, um die markierten Spermien zu inaktivieren.

Auch die WO 2006/012597 A2 betrifft die geschlechtsspezifische Markierung von Spermien und die Inaktivierung einer markierten Population durch Erwärmung. DNA-Sequenzen zur geschlechtsspezifischen Markierung von Spermien werden nicht angegeben.

Die WO 2007/095279 beschreibt die spezifische Markierung Leukämie spezifischer Blutzellen durch magnetische Anreicherung, nachdem die Zellen mit Magnetpartikeln kontaktiert wurden, die ein Zelltyp spezifisches Oligonukleotid mit vorgegebener Sequenz tragen.

Die DE 69905832 T2 beansprucht eine für die Durchflusszytometrie geeignete Zusammensetzung, die Kern aus einem Halbleiter- Nanokristall mit einer äußeren Schicht, die einen Liganden umfasst, und ein mit dem Kern verbundenen ersten Teil eines Bindepaares.

Medarova et al, (Nature Medicine 372-377 (2007)) beschreiben magnetische Nanopartikel, die vollflächig gebundene siRNAs und Myristyl- Polyarginin - Peptide zur Penetrationsverstärkung enthalten, um in Krebszellen durch Kernresonanzanalyse den Eintritt der siRNA an Tumorzellen sichtbar zu machen.

Die DE 10 2005 044530 beschreibt ein Verfahren zum Sortieren von Säugerspermatozoen durch Anfärbung der DNA mit einem Fluoreszenzfarbstoff, beispielsweise Hoechst Bisbenzimid H 33342, Ausrichten der Spermatozoen in einer elektrisch leitfähigen isotonischen Hüllflüssigkeit, Vereinzeln der Spermatozoen in Tropfen der Hüllflüssigkeit, Hindurchtretenlassen der Tropfen durch den Lichtstrahl eines Anregungslasers und Messen der relativen Fluoreszensintensität jedes Spermatozoons, und Auftrennen der Spennatozoen enthaltenden Tropfen entsprechend der gemessenen Fluoreszensintensität. Zur Verbesserung des Sortiererfolgs werden die Spermatozoen durch Fluorid im Medium immobilisiert, was deren Ausrichtung erleichtert.

Garner et al., Theriogenology 65 (2006) 943-957, beschreiben klassische Hybridisierungsverfahren unter Verwendung von Hoechst 33342, einem gängigen Fluoreszenzfarbstoff zur quantitativen Einfärbung von DNA.

Die US 5,840,504 beschreibt die Verwendung von Antikörpern, die gegen geschlechtschromosomenspezifische Oberflächenantigene von Spermien gerichtet sein sollen, zur Identifikation und Selektion X-chromosomenhaltiger und Y-chromosomenhaltiger Spermatozoen.

Bei den bekannten Verfahren zur Sortierung von Spermatozoen ist nachteilig, dass die Spermatozoen für die optische Detektion ausgerichtet sein müssen, um die quantitativen Unterschiede des Fluoreszenzsignals für X-Chromosomen-haltige bzw. Y-Chromosomenhaltige Spermatozoen messen zu können.

### Aufgabe der Erfindung

Gegenüber dem bekannten Stand der Technik ist es daher Aufgabe der Erfindung, ein alternatives Verfahren zur Herstellung von geschlechtschromosomspezifischen Spermafraktionen bereitzustellen, sowie für das Herstellungsverfahren verwendbare Stoffe und Herstellungsverfahren für diese bereitzustellen.

### Allgemeine Beschreibung der Erfindung

Die Erfindung löst die vorgenannten Aufgaben durch Bereitstellen von Detektionskonjugaten, die eine geschlechtschromosomspezifische Nukleinsäuresequenz enthalten, und eine detektierbare Markierung enthalten, sowie ein Sortierverfahren unter Verwendung der Detektionskonjugate zur Herstellung geschlechtschromosomspezifischer Präparate oder Fraktionen. Die geschlechtschromosomspezifische Nukleinsäuresequenz ist vorzugsweise für das Y-Chromosom spezifisch und ist eine einzelsträngige RNA, DNA oder vorzugsweise PNA (Peptid - Nukleinsäure, bei der die (desoxy-) Ribose - Phosphatkette von DNA bzw. RNA durch ein Polypeptid, insbesondere aus N-(2-Aminoethyl)-Glycin-Einheiten in Peptidbindung ersetzt ist, das die Basen trägt).

Die detektierbare Markierung des Detektionskonjugats besteht in einer ersten Ausführungsform aus einem an der Nukleinsäuresequenz gebundenen Fluorochrom, und einem in einem Abstand zum Fluorochrom an der Nukleinsäure gebundenen Quencher. Beispielsweise ist das Fluorochrom an einem ersten Ende der geschlechtschromosomspezifischen Nukleinsäuresequenz angeordnet, und der Quencher, der die von dem Fluorochrom emittierte Strahlung im Wesentlichen aufnimmt und strahlungslos abgibt, ist am gegenüberliegenden zweiten Ende der Nukleinsäuresequenz angeordnet, oder in einer zweiten Ausführungsform aus einem metallischen Nanopartikel, vorzugsweise einem Goldnanopartikel, der vorzugsweise ohne zusätzliches Kopplungsreagenz, d.h. unmittelbar, an die Nukleinsäuresequenz gebunden ist. Daher kann dieses Detektionskonjugat aus einem Oligonukleotid, das DNA, RNA oder PNA ist, einem an dem Oligonukleotid gebundenen Fluorochrom und einem vom Fluorochrom beabstandet an dem Oligonukleotid gebundenen Quencher bestehen, wobei das Oligonukleotid DNA, RNA oder PNA ist und eine geschlechtschromosomspezifische oder eine allel- oder SNP-spezifische Nukleinsäuresequenz aufweist.

Bei beiden Ausführungsformen der Erfindung erfolgt die geschlechtschromosomspezifische Färbung und Detektion von Spermatozoen dadurch, dass sich die geschlechtschromosomspezifische Nukleinsäuresequenz des Detektionskonjugats an das Geschlechtschromosom anlagert und dadurch das detektierbare Signal der Markierung verändert. In der ersten Ausführungsform kann ein Fluoreszenzsignal vom Fluorochrom nur detektierbar abgestrahlt werden, wenn das Detektionskonjugat mittels seiner Nukleinsäuresequenz mit dem Geschlechtschromosom hybridisiert, wobei das Fluorochrom vom Quencher getrennt wird. Im Unterschied zur Hybridisierung mit DNA der Zielzelle ist das Detektionskonjugat der ersten Ausführungsform im nicht mit einem (Geschlechts-) Chromosom hybridisierten Zustand in einer solchen Konformation, dass der Quencher innerhalb des Förster-Radius des Fluorochroms ist und die vom Fluorochrom emittierte Strahlung aufnimmt und im Wesentlichen strahlungsfrei, beispielsweise durch Dissipation abgibt. Entsprechend ist ohne spezifische Hybridisierung ein Fluoreszenzsignal des Detektionskonjugats dieser Ausführungsform nicht oder nur signifikant reduziert detektierbar.

Für die Zwecke der Erfindung bedeutet die Bezugnahme auf eine Nukleinsäuresequenz ein Oligonukleotid, insbesondere in Form von PNA, das vorzugsweise eine mit einem vorbestimmten genomischen DNA-Abschnitt einer zu analysierenden Zielzelle hybridisierbare Basenfolge, insbesondere eine geschlechtschromosomspezifische Basenfolge aufweist.

In der zweiten Ausführungsform ändert sich durch die geschlechtschromosomspezifische Hybridisierung der Nukleinsäuresequenz des Detektionskonjugats die Oberflächenplasmonresonanz. Daher ändert sich auch in dieser Ausführungsform das detektierbare Signal abhängig von der geschlechtschromosomspezifischen Hybridisierung der Nukleinsäuresequenz des Detektionskonjugats mit dem Geschlechtschromosom der analysierten Zielzelle, die insbesondere Gameten sind, besonders bevorzugt Spermatozoen.

In der zweiten Ausführungsform kann der Bindeanteil ein Antikörper sein, natürlich oder synthetisch, einkettig oder zweikettig, vorzugsweise eine Nukleinsäuresequenz, beispielsweise RNA, DNA, vorzugsweise PNA, oder ein Rezeptor-spezifischer Ligand oder eine andere Verbindung, die spezifisch mit einem oberflächengebundenen Bestandteil einer Zelle oder einem zellinternen Bestandteil in Wechselwirkung tritt, insbesondere ein Antikörper. Dieses Detektionskonjugat kann aus einem an einen Metallnanopartikel gebundenen Oligonukleotid bestehen, wobei das Oligonukleotid DNA, RNA oder PNA ist und eine geschlechtschromosomspezifische oder eine allel- oder SNP-spezifische Nukleinsäuresequenz aufweist.

Die Erfindung der zweiten Ausführungsform wird auch mit Bezug auf Detektionskonjugate beschrieben, die als Bindeanteil eine Nukleinsäuresequenz, vorzugsweise PNA enthalten, wobei die Nukleinsäuresequenz stellvertretend für andere Bindeanteile genannt ist und durch andere Bindeanteile, insbesondere Antikörper ersetzt sein kann. Entsprechend wird bezüglich der zweiten Ausführungsform die Nukleinsäuresequenz stellvertretend für andere Bindemoleküle im Detektionskonjugat verwendet, wobei die Nukleinsäuresequenz entsprechend durch andere Bindemoleküle ersetzt sein kann.

Überraschenderweise ist die Hybridisierung der geschlechtschromosomspezifschen Nukleinsäuresequenz des Detektionskonjugats auch mit der hoch kondensierten DNA möglich, wie sie in Spermatozoen vorliegt. Eine solche Hybridisierung konnte aus dem Stand der Technik nicht abgeleitet werden, da dort interkalierende Farbstoffe, insbesondere Höchst Bisbenzimid 333425 eingesetzt werden, die kleiner als das erfindungsgemäße Detektionskonjugat sind und sich ohne Sequenzspezifität an DNA anlagern.

Es bevorzugt, dass in der zweiten Ausrührungsform die Nanopartikel des Detektionskonjugats gebundene penetrationsverstärkende Verbindungen aufweisen, beispielsweise Polyargininpeptide, besonders bevorzugt mit einem gebundenen Myristinsäurerest.

Zur Herstellung geschlechtschromosomspezifischer Spermafraktionen kann in beiden Ausführungsformen der Erfindung das folgende Verfahren durchgeführt werden:
Kontaktieren intakter, lebensfähiger Gameten, insbesondere Spermatozoen, die von einem männlichen, nicht-menschlichen Säugetier erhalten wurden, mit einem Detektionskonjugat, Vereinzeln der Gameten, insbesondere Spermatozoen, entweder in Tropfen einer Hüllflüssigkeit, die vorzugsweise elektrisch leitfähig und isotonisch ist, oder in einem Fluidstrom, der beispielsweise in einem Durchflusszytometer erzeugt wird, Anregen des Detektionskonjugats, z.B. durch Einstrahlen von Strahlung einer Anregungswellenlänge, in der ersten Ausführungsform zur Anregung der Fluoreszenz, in der zweiten Ausführungsform zur Anregung der Oberflächenplasmonresonanz, Detektion des von dem Detektionskonjugat abgegebenen Signals, in der ersten Ausführungsform durch Messen der Fluoreszenz, in der zweiten Ausführungsform durch Messen der abgegebenen Strahlung der Oberflächenplasmonresonanz, Sortieren der Gameten, insbesondere Spermatozoen, entsprechend der gemessenen Signalintensität zur Herstellung zumindest zweier Fraktionen, beispielsweise mit einer Signalintensität auf einer Seite eines Schwellenwerts für die geschlechtschromosomspezifische Gameten-, insbesondere Spermatozoenfraktion, für deren Geschlechtschromosom die Nukleinsäuresequenz des Detektionskonjugats hybridisierbar bzw. spezifisch war, und einer Fraktion der Gameten, insbesondere Spermatozoen, für die Signalintensitäten auf der anderen Seite eines Schwellenwerts gemessen wurden, bei denen entsprechend die Nukleinsäuresequenz des Detektionskonjugats nicht hybridisiert.

Ein besonderer Vorteil des erfindungsgemäßen Verfahrens liegt darin, dass die verwendeten Detektionskonjugate jeweils spezifisch für das in einem Gameten, insbesondere Spermatozoon enthaltene Geschlechtschromosom ein signifikant qualitativ abweichendes detektierbares Signal bei entsprechender Anregung emittieren, und das Signal abhängig von der Hybridisierung signifikant abweicht, und diese Abweichung hinreichend groß ist, dass sie ohne eine spezifische Orientierung der Spermatozoen gegenüber der eingestrahlten Anregungsenergie bzw. gegenüber dem Detektor zur Aufnahme des emittierten Signals gemessen werden kann. Für die Zwecke der Beschreibung wird der Begiff Spermatozoon als ein bevorzugtes Beispiel für Gameten verwendet.

Entsprechend kann das erfindungsgemäße Verfahren zur Herstellung geschlechtschromosomspezifischer Spermafraktionen vorzugsweise mit der Vereinzelung der Spermatozoen während der Detektion eines Signals vom Detektionskonjugat und während der anschließenden Sortierung in Fraktionen auf Basis des gemessenen Detektionssignals erfolgen, auch ohne Spermatozoen entlang ihrer Längsachse auszurichten, z.B. in einem Durchflusszytometer mit kontinuierlicher Flüssigkeitsphase.

Vorzugsweise werden die vereinzelten Spermatozoen im Anschluss an die Detektion des Signals des Detektionskonjugats einer Fraktion zugeordnet, beispielsweise durch Ablenkung von Tropfen oder Volumenabschnitte der Hüll- oder Trägerflüssigkeit, die die Spermatozoen enthalten. Die Ablenkung kann z.B. durch ein vom detektierten Signal abhängig erzeugtes elektrisches Feld erfolgen. Alternativ zu dieser Sortierung in zumindest zwei Fraktionen ist es auch möglich, die vereinzelten Spermatozoen im Anschluss an die Detektion in Abhängigkeit von der Höhe des Detektionssignals unbeeinflusst in dem Trägermedium bzw. der Hüllflüssigkeit zu belassen, bzw. abhängig von dem Detektionssignal einen Anteil der Spermatozoen zu inaktivieren, beispielsweise durch Erwärmen mittels gezielter Laserbestrahlung der Spermatozoen, die in der vorangegangenen Detektion einen Schwellenwert des Signals über- oder unterschritten haben. Bei dieser Variante beider Ausführungsformen enthält eine erzeugte Spermatozoenfraktion die nicht inaktivierten, d.h. z.B. nicht bestrahlten Spermatozoen, und inaktivierte (nicht befruchtungsfähige) Spermatozoen, wobei die Inaktivierung vom Unterschreiten über Überschreiten eines Schwellenwerts für das gemessene Detektionssignal abhängt. Ein entsprechendes Durchflusszytometer kann daher einen Laser aufweisen, der zur inaktivierenden Bestrahlung einzelner Zellen im kontinuierlichen Flüssigkeitsstrom in Abhängigkeit von dem Signal eingerichtet ist, das vom Detektionskonjugat emittiert wird.

Vorzugsweise werden die Spermatozoen mit dem Detektionskonjugat in Mischung mit einem Penetrationshilfsmittel kontaktiert, um die Aufnahme des Detektionskonjugats in die Spermatozoen zu erleichtern. Geeignete Penetrationshilfsmittel sind für tierische Zellen verwendbare Transfektionsagenzien, z.B. Fugene, Lipofectamine, Oligofectamine, Optifect, DMRIE C, AntHD, Penetratin 43-58, HIV 1-Tat Protein, Tat, Peptide 49-59, Tat, Peptide 48-62, Tat 2-4, Tat, Peptide (YGRKKRRQRRRGYGRKKRRQRRRG), amphipathische Peptide (MAPs), z.B. der Aminosäuresequenz KALA oder KLAL, cis-y-amino-L-Prolin-haltige Peptide, VP22, Galparan, Transportan, MPG, SynB1, Fushi-tarazu, Engrailed, pVEC, plsl, Hoechst 33342. Weiterhin können die Detektionskonjugate, insbesondere der ersten Ausführungsform zur Verstärkung der Penetration als Liposomen formuliert sein, für die zweite Ausführungsform bevorzugt in Mischung mit Liposomen formuliert.

Eine bevorzugte, für das Y-Chromosom insbesondere des Rindes spezifische Nukleinsäuresequenz ist: 5' agc aca tct-cgg tcc ctg 3' (Seq.-ID Nr. 1), ggc gac tgt gca agc aga (Seq.-ID Nr. 2), aga gac tgt gga acc gg (Seq.-ID Nr. 3), ggc gat tgt tca acc ag (Seq.-ID Nr. 4), und zu diesen jeweils komplementäre Nukleinsäuresequenzen und Abschnitte von 10 bis 30, vorzugsweise von 15 bis 20 Nukleotiden der Sequenz Y1.2, die unter der Zugangsnummer M26067 bei GenBank zugänglich ist, oder die gesamte Sequenz Y1.2 und zu diesen jeweils komplementäre Nukleinsäuresequenzen.

Alternativ zur geschlechtschromosomspezifischen Nukleinsäuresequenz kann ein erfindungsgemäßes Detektionskonjugat eine für ein Allel oder ein SNP (Einzelnukleotid-Polymorphismus) spezifische Nukleinsäuresequenz enthalten, um Zellen, insbesondere Spermatozoen, allelspezifisch bzw. SNP-spezifisch zu fraktionieren.

Für die erste Ausführungsform geeignete Fluorochrome können aus der Gruppe ausgewählt werden, die aus Cy3, PE, FITC, APC, Alexa-Farbstoffen und Atto-Farbstoffen besteht, geeignete Quencher aus der Gruppe, die aus Dabcyl, Dabsyl, Dark-Hole-Quencher, Black-Berry-Quencher und QSY-Farbstoffen besteht.

In der zweiten Ausführungsform der Erfindung ist es bevorzugt, dass die Detektionskonjugate aus kolloidalen Goldnanopartikeln mit unmittelbar daran gebundenen geschlechtschromosomspezifischen Nukleinsäuresequenzen bestehen, optional zusätzlich mit unmittelbar am Goldnanopartikel gebundenen penetrationsverstärkenden Verbindungen. Diese Detektionskonjugate weisen daher die geschlechtschromosomspezifische Nukleinsäuresequenz in unmittelbarer Bindung an kolloidale Metallnanopartikel, insbesondere kolloidale Goldnanopartikel auf, gegebenenfalls zusätzlich unmittelbar an den Nanopartikel gebundene penetrationsverstärkende Agenzien.

Für die Anregung der Detektionskonjugate mit kolloidalem Goldnanopartikel kann beispielsweise Licht mit einer Wellenlänge von 450 bis 600 nm, bevorzugt 480 bis 540 nm, besonders bevorzugt etwa 520 nm zur Bestrahlung verwendet werden. Als Signal für die geschlechtschromosomspezifische Detektion wird die Absorption der Anregungsstrahlung gemessen, wobei vorzugsweise die geschlechtschromosomspezifische Hybridisierung der Nukleinsäuresequenz eines Detektionskonjugats als Veränderung der Absorption detektierter ist, gegebenenfalls durch Verschiebung der Wellenlänge, insbesondere zu höheren Wellenlängen.

Aufgrund der Detektion der vom Detektionskonjugat emittierten Strahlung, in der ersten Ausführungsform als Fluoreszenz, in der zweiten Ausführungsform als Fluoreszenz, Absorption oder Wellenlängenverschiebung, ermöglichen beide Ausführungsformen der Erfindung ein Sortierverfahren für Zellen, insbesondere für Spermatozoen, mit den Schritten der berührungslosen Detektion der geschlechtschromosomspezifischen Hybridisierung und der anschließenden Fraktionierung und/oder Inaktivierung vereinzelter Spermatozoen in Abhängigkeit von dem detektierten Signal.

Bevorzugt ist bei der ersten Ausführungsform die Nukleinsäuresequenz kovalent mit dem Fluorochrom und dem Quencher verbunden, beispielsweise durch eine unmittelbare chemische Bindung zwischen Nukleinsäuresequenz und Fluorochrom bzw. Quencher. Die chemische Bindung kann eine Amid-, Thioether- oder Esterbindung sein.

Die Detektionskonjugate der zweiten Ausführungsform sind vorzugsweise kolloidale Goldnanopartikel, die mit geschlechtschromosomspezifischer PNA dadurch konjugiert sind, dass Nanopartikel mittels eines Ultrakurzpulslasers von Gold in wässrigen Medium abgetragen werden und in dem wässrigen Medium die Nukleinsäuresequenz, optional zusätzlich gleichzeitig oder später zugegeben penetrationsverstärkendes Agenz vorliegt. Die Erzeugung der Goldnanopartikel durch Laserabtragung mittels Ultrakurzpulsen stellt Nanopartikel mit reaktiver Oberfläche her, die auch partielle oxidierte Au+, Au3+ an der Oberfläche aufweisen können. Überraschenderweise ist gefunden worden, dass allein die Erzeugung von Metallnanopartikeln durch Ultrakurzpuls-Laserabtragung in Anwesenheit der geschlechtschromosomspezifischen Nukleinsäuresequenz, gegebenenfalls auch für in Mischung oder später zugegebene penetrationsverstärkende Agenzien eine unmittelbare Bindung der Nukleinsäuresequenz bzw. des penetrationsverstärkenden Agenzes mit dem Goldnanopartikel ergibt. Durch die Ultrakurzpuls-Laserabtragung werden die Metallpartikel, insbesondere Goldnanopartikel, partiell oxidiert und wirken als Elektronenakzeptoren, die mit Bindeanteilen, insbesondere Nukleinsäuresequenzen, und mit wahlweise gleichzeitig oder später anwesenden penetrationsverstärkenden Agenzien eine Bindung eingehen, z.B. eine Komplexbindung oder eine koordinative Bindung.

Vor Erhöhung der Bindungsstärke können die Nukleinsäuresequenzen bzw. das penetrationsverstärkende Agenz mit mit Gold reaktiven Gruppen versehen sein, insbesondere mit Thiol-, Carboxy-, Amid- und/oder Amingruppen, die die Nukleinsäuresequenzen am 3'- oder 5'-Ende zur Bindung an einen Nanopartikel tragen, vorzugsweise am 3'-Ende. Es ist möglich, dieses Herstellungsverfahren kontinuierlich in einer Durchflusskammer durchzuführen, wobei wässriges Medium mit einem Gehalt an Nukleinsäuresequenz am Gold wobei strömen gelassen wird, während von dem Gold kolloidale Nanopartikel durch Einstrahlung von Ultrakurzpulslaserstrahlung hergestellt werden. In dieser Ausführungsform können penetrationsverstärkende Agenzien in einem gewünschten Verhältnis in Mischung mit Nukleinsäuresequenzen eingesetzt werden, oder penetrationsverstärkende Agenzien können stromabwärts des Ortes der Erzeugung der kolloidalen Goldnanopartikel dem Fluidstrom zugegeben werden, sodass nach Reaktion der Nanopartikel mit Nukleinsäuresequenzen reaktive Stellen der Nanopartikel mit penetrationsverstärkendem Agenz reagieren können.

Es können magnetische Nanopartikel erzeugt werden, z.B. Goldnanopartikel, die dann als ein Aspekt der zweiten Ausführungsform durch Detektion der Verschiebung der Relaxation bei Kopplung an das spezifische Geschlechtschromosom detektierbar sind, z.B. durch Detektion des Relaxationsunterschieds auf Grund der spezifischen Bindung des Detektionskonjugats an oder in Säugerspermatozoen, z.B. des Relaxationsunterschieds zwischen X-Chromosom - haltigen Spermatozoen gegenüber Y-Chromosomen-haltigen bei geschlechtschromomspezifischer Nukleinsäuresequenz zur anschließenden Selektion der Spermatozoen. Bei unspezifischer Nukleinsäuresequenz des Detektionskonjugats kann die Detektion und Selektion auf Basis des quantitativen Relaxationsunterschieds erfolgen, da sich Spermatozoen auch durch den unterschiedlichen Gesamt-DNA-Gehalt unterscheiden.

Alternativ zur geschlechtschromosomspezifischen Nukleinsäuresequenz kann eine willkürliche Nukleinsäuresequenz oder ein unspezifisch an DNA bindender Farbstoff, z.B. Hoechst Bisbenzimid H33342 in den Detektionskonjugaten enthalten sein, sodass zur Identifikation anhand des Geschlechtschromosoms ein quantitativer Unterschied des Signals wegen des geringeren Gesamt- DNA-Gehalts der Y-chromosomenhaltigen Spermatozoen zu detektieren ist.

Die Nanopartikel, die in erfindungsgemäßen Detektionskonjugaten enthalten sind, sind vorzugsweise hergestellt durch Ultakurzpulslaserabtragung eines Metalls im wässrigen Milieu, z.B. eingetaucht in eine wässrige Zusammensetzung, wobei der Ultrakurzpuls eine Pulsdauer von 10 fs bis 15 ps hat, bei einer Wellenlänge von größer 330 nm und maximal 1030 nm, insbesondere im Bereich von 500 bis 1000 nm. Die Zeitdauer der Abtragung beträgt vorzugsweise ca. 10 bis 200 s, z.B. 40 bis 60s, insbesondere 53 s, bei einer Pulsenergie von ca 50 bis 200 µJ, insbesondere 80 bis 120 µJ, vorzugsweise 120 µJ, die Pulsdauer ca 100 bis 140 fs, insbesondere ca. 120 fs, vorzugsweise bei 800 nm.

Dieses Herstellungsverfahren ergibt Nanopartikel, die auch mit gebundenen Bindeanteil, das z.B. ein Peptid oder eine geschlechtschromosomenspezifischen Nukleinsäuresequenz, vorzugsweise als PNA ist, eine für die Penetration der Zellwand von Säugerspermatozoen, insbesondere des Rindes, besonders geeignete Größe und/oder Konformation haben. Die Nanopartikel haben z.B. eine Größe von 1 bis 150 nm, bis 100 nm, vorzugsweise 5 bis 50 oder bis 25 nm.

Das Herstellungsverfahren für Detektionskonjugate mit einem Gehalt an Nanopartikeln erzeugt wegen der Erzeugung von Nanopartikeln mittels Ultrakurzpulslaserstrahlung Detektionskonjugate mit besonders geringer Nanopartikelgröße, da die Nanopartikel in einer sehr geringen Zeitspanne, z.B. von 1 bis 10 ps eine sehr geringe Größe aufweisen, in der eine hohe Reaktivität des Goldnanopartikels vorliegt, beispielsweise mit Thiol-haltigen Nukleinsäuresequenzen, während im Anschluss an diese Zeitspanne die Agglomeration der Nanopartikel einsetzt. Entsprechend weisen die für das Herstellungsverfahren einzusetzenden Nukleinsäuresequenzen vorzugsweise Thiol-, Keto-, Carboxy-, Amid- oder Amingruppen auf, um eine entsprechende koordinative Bindung zum Nanopartikel herzustellen, d.h. ohne die Verwendung eines zusätzlichen Kopplungsreagenzes zwischen Nukleinsäuresequenz und Nanopartikel.

Bevorzugt stammt das nicht-menschliche Sperma vom Paarhufer oder Unpaarhufer, insbesondere vom Rind, Schwein, Schaf, Kamel oder Pferd.

### Genaue Beschreibung der Erfindung

Die Erfindung wird nun genauer anhand von Beispielen beschrieben.

### Beispiel 1: Herstellung eines Detektionskoniugats

Entsprechend der ersten Ausführungsform der Erfindung wurde ein Detektionskonjugat durch kovalente Verbindung eines Fluorochroms, beispielsweise Cy3, PE, FITC, APC, eines Alexa-Farbstoffs oder eines Atto-Farbstoffs an das 5'-Ende einer für das Y-Chromosom spezifischen Nukleinsäuresequenz gebunden, und an das 3'-Ende der Nukleinsäuresequenz ein zur Absorption der vom Fluorochrom geeigneter Quencher, beispielsweise Dabcyl, Dabsyl, Dark-Hole-Quencher, Black-Berry-Quencher, oder ein QSY- Farbstoff. Die Nukleinsäuresequenz war ein PNA- Oligonukleotid der Sequenz: 5' agc aca tct cgg tcc ctg 3'

### Beispiel 2: Herstellung eines Detektionskoniugats mit Nanogoldpartikel

Zur Herstellung eines Detektionskonjugats mit einem metallischen Nanopartikel wurde Goldfolie in eine wässrige Lösung eingebracht, die die in Beispiel 1 verwendete Nukleinsäuresequenz enthielt. Die Goldfolie wurde mit 120 fs Laserimpulsen bei einer Wellenlänge von 800 nm bei einer maximalen Energie von 400 µJ pro Puls, Strahldurchmesser 4 mm bei einem Abstand von etwa 40 mm von der Linse zur Goldfolie bei einer Wiederholungsrate von 5 kHz bestrahlt. Der Energieeintrag auf die Goldfolie war ca. 100 µJ. Die wässrige Lösung enthielt ca. 3 µM Nukleinsäuresequenz in Wasser, mit einer Schichthöhe von ca. 1 cm oberhalb der Goldfolie.

Die Analyse der Reaktionsprodukte durch Polyacrylamidgelelektrophorese zeigte eine nur geringe Degradation der Nukleinsäuresequenz. Die Analyse der Reaktionsprodukte durch Transmissions- Elektronenmikroskopie ergab, dass die Konjugate eine Größenverteilung mit einem Mittel von ca. 5,2 bis 5,5 nm aufwiesen. Die Konjugate waren nicht agglomeriert und zeigten eine etwa sphärische Gestalt bei den verwendeten Parametern wurden ca. 20 µg/min Goldpartikel erzeugt, die ohne weitere chemische Kopplungsreagenzien eine stabile Bindung mit der Nukleinsäuresequenz eingingen.

### Beispiel 3: Detektion Y-Chromosomen-haltiger Spermien in Frischsamen und geschlechtsspezifische Sortierung

Frisch gewonnener Bullensamen wurde in üblicher Weise in Verdünner verdünnt und mit Detektionskonjugat, das nach Beispiel 1 oder Beispiel 2 hergestellt wurde, 30 bis 60 min bei einer Temperatur von 20 °C bis 40 °C inkubiert und anschließend in einem Durchflusszytometer gemäß US 5125759 oder der DE 10 2005 044 530 mit Licht der jeweiligen Anregungswellenlänge für das Fluorochrom bzw. für 520 nm für die Goldnanopartikel bestrahlt. Die jeweilige Emission wurde gemessen.

Für die mit Detektionskonjugat nach Beispiel 1 spezifisch markierten Y-Chromosom-haltigen Spermatozoen wurde ein Fluoreszenzsignal gemessen, während die X-Chromosom-haltigen Spermatozoen kein Fluoreszenzsignal emittierten. Dies zeigt, dass dieses Detektionskonjugat nur bei Hybridisierung der Nukleinsäuresequenz ein detektierbares Signal unter Bestrahlung bei der Anregungswellenlänge erzeugt, während Zellen, die keine mit der Nukleinsäuresequenz des Detektionskonjugats hybridisierende Nukleinsäure enthalten, kein oder nur ein unbedeutendes Fluoreszenzsignal unter Bestrahlung erzeugen.
Für die mit Detektionskonjugat nach Beispiel 2 chromosomenspezifisch angefärbten Spermatozoen wurde eine Veränderung der detektierten Oberflächenplasmonresonanz für die Y-Chromosom-haltigen Spermatozoen festgestellt, während die X-Chromosom-haltigen Spermatozoen eine signifikant weniger veränderte Oberflächenplasmonresonanz zeigten.

Abhängig von dem detektierten Signal wurden die Spermatozoen durch ein elektrisches Feld in geschlechtschromosomspezifische Fraktionen abgelenkt.

Alternativ zur Verwendung eines Durchflusszytometers, d.h. ohne Ausrichtung der Spermatozoen in eine bestimmte Position bezüglich der Längsachse konnte das Detektions- und Sortierverfahren auch in einem herkömmlichen Durchflusszytometer mit Einrichtung zur signalabhängigen Zellsortierung (BD Bioscience, FACScalibur oder FACSAria) durchgeführt werden.

Optional wurde ein Fluorid zur Immobilisierung der Spermatozoen zugesetzt, z.B. in die während des Sortierverfahrens verwendete Hüll- oder Transportflüssigkeit, und/oder vor oder während des Zusatzes des Detektionskonjugats, um die Penetration des Detektionskonjugats in die Spermatozoen zu erhöhen. Fluoridionen wurden im Bereich von 0,1 bis 100 mM, vorzugsweise von 10 nM bis 10 mM zugesetzt. Es wurde gefunden, dass die optimale Konzentration des Fluorids, z.B. NaF oder KF, zwischen verschiedenen Spezies und für Individuen abwich. Die optimale Konzentrationen für die Spezies ist spezifisch und konnte allgemein als die Konzentration bestimmt werden, die bei mikroskopischer Betrachtung eine Immobilisierung von wenigstens 90% der Spermatozoen ergab, vorzugsweise von im wesentlichen allen Spermatozoen. Entsprechend bezieht sich die vorliegende Erfindung auch auf Zusammensetzungen der mit dem erfindungsgemäßen Verfahren hergestellten Spermafraktionen, und auf Verfahren zur Herstellung von geschlechtsspezifischen Spermafraktionen und anschließenden Konservierung der Spermafraktionen von nicht-menschlichen Säugetieren, jeweils vorzugsweise in Anwesenheit von Fluorid und/oder Antioxidationsmitteln.

## Patentansprüche

1. Verfahren zur Herstellung einer Fraktion von nicht-menschlichen Säugergameten mit einem überwiegenden Anteil befruchtungsfähiger X-Chromosomen-haltiger oder Y-Chromosomen-haltiger Gameten durch Detektion der von einem Detektionskonjugat unter Einstrahlung von Strahlung mit einer Anregungswellenlänge emittierten Strahlung, **dadurch gekennzeichnet, dass** das Detektionskonjugat ein Oligonukleotid mit einer geschlechtschromosomspezifischen Nukleinsäuresequenz mit einem gebundenen Fluorochrom und einen in einem Abstand zum Fluorochrom gebundenen Quencher aufweist.

2. Verfahren zur Herstellung einer Fraktion von nicht-menschlichen Säugergameten mit einem überwiegenden Anteil befruchtungsfähiger X-Chromosomen-haltiger oder Y-Chromosomen-haltiger Gameten durch Detektion der von einem Detektionskonjugat unter Einstrahlung von Strahlung mit einer Anregungswellenlänge emittierten Strahlung, **dadurch gekennzeichnet, dass** das Detektionskonjugat ein Oligonukleotid mit einer geschlechtschromosomspezifischen Nukleinsäuresequenz aufweist, die unmittelbar an einen Goldnanopartikel gebunden ist.

3. Verfahren nach Anspruch 2, **dadurch gekennzeichnet, dass** der Goldnanopartikel eine Größe von 1 bis 100 nm hat.

4. Verfahren nach Anspruch 2 oder 3, **dadurch gekennzeichnet, dass** das Detektionskonjugat erhältlich ist durch Erzeugung von Goldnanopartikeln durch Ultrakurzpulslaserabtragen von Gold in einer Flüssigkeit, die die Nukleinsäuresequenz enthält.

5. Verfahren nach Anspruch 4, **dadurch gekennzeichnet, dass** der Ultrakurzlaserpuls eine Pulsdauer von 10 fs bis 15 ps bei einer Wellenlänge von mindestens 330 nm bis maximal 1030 nm aufweist.

6. Verfahren nach einem der Ansprüche 2 bis 4, **dadurch gekennzeichnet, dass** die Abtragszeit 10 bis 200 s bei einer Pulsenergie von 50 bis 200 µJ und einer Pulsdauer 100 bis 140 fs bei 800 nm beträgt.

7. Verfahren nach einem der Ansprüche 2 bis 6, **dadurch gekennzeichnet, dass** die Nukleinsäuresequenz zumindest eine reaktive Gruppen enthält, die ausgewählt ist aus der Gruppe, die Ketogruppen, Amingruppen, Thiolgruppen, und Carboxylgruppen enthält.

8. Verfahren nach einem der voranstehenden Ansprüche, **dadurch gekennzeichnet, dass** das Oligonukleotid PNA ist.

9. Verfahren nach einem der voranstehenden Ansprüche, **dadurch gekennzeichnet, dass** die Nukleinsäuresequenz die Basenfolge 5' agc aca tct cgg tcc ctg 3' (Seq.-ID Nr. 1) oder eine dazu komplementäre Basenfolge aufweist.

10. Verfahren nach einem der voranstehenden Ansprüche, **dadurch gekennzeichnet, dass** Spermatozoen in Abhängigkeit von dem gemessenen Signal in eine von zumindest zwei Fraktionen gelenkt werden.

11. Verfahren nach einem der Ansprüche 1 bis 8, **dadurch gekennzeichnet, dass** Gameten in Abhängigkeit von dem detektierten Signal durch gezielte Laserbestrahlung wärmebehandelt werden.

12. Verfahren nach einem der voranstehenden Ansprüche, **dadurch gekennzeichnet, dass** die Fraktion von nicht-menschlichen Säugergameten intakt und lebensfähig ist.

13. Verfahren nach einem der voranstehenden Ansprüche, **dadurch gekennzeichnet, dass** die Säugergameten einen Gehalt an Fluorid und/oder Antioxidationsmittel aufweist.

14. Verfahren nach einem der voranstehenden Ansprüche, **dadurch gekennzeichnet, dass** die Gameten Spermatozoen sind.

15. Fraktion nicht-menschlicher Säugergameten mit einem überwiegenden Anteil befruchtungsfähiger X-Chromosomen-haltiger oder Y-Chromosomen-haltiger Gameten, erhältlich durch ein Verfahren nach einem der voranstehenden Ansprüche und mit einem Gehalt an einem Detektionskonjugat, das ein Oligonukleotid mit einer geschlechtschromosomspezifischen Nukleinsäuresequenz mit einem gebundenen Fluorochrom und in einem Abstand zum Fluorochrom gebundenen Quencher aufweist.

16. Fraktion nicht-menschlicher Säugergameten mit einem überwiegenden Anteil befruchtungsfähiger X-Chromosomen-haltiger oder Y-Chromosomen-haltiger Gameten, erhältlich durch ein Verfahren nach einem der Ansprüche 1 bis 14 und mit einem Gehalt an einem Detektionskonjugat, das ein Oligonukleotid mit einer geschlechtschromosomspezifischen Nukleinsäuresequenz aufweist, die an einen Goldnanopartikel gebunden ist.

17. Fraktion nach einem der Ansprüche 15 oder 16, **dadurch gekennzeichnet, dass** die Gameten Spermatozoen sind.

## Claims

1. Method for producing a fraction of nonhuman mammalian gametes having a predominant portion of fertile gametes containing an X-chromosome or a Y-chromosome by detection of the radiation emitted by a detection conjugate at irradiation with radiation having an excitation wavelength, **characterized in that** the detection conjugate has an oligonucleotide having a sex-chromosome-specific nucleic acid sequence having a bound fluorochrome and having a quencher bound at a distance from the fluorochrome.

2. Method for producing a fraction of nonhuman mammalian gametes having a predominant portion of fertile gametes containing an X-chromosome or a Y-chromosome by detection of the radiation emitted by a detection conjugate at irradiation with radiation having an excitation wavelength, **characterized in that** the detection conjugate has an oligonucleotide having a sex-chromosome-specific nucleic acid sequence that is bound directly to a gold nanoparticle.

3. Method according to claim 2, **characterized in that** the gold nanoparticle has a size of from 1 to 100 nm.

4. Method according to claim 2 or 3, **characterized in that** the detection conjugate is obtainable by the generation of gold nanoparticles by ultrashort pulse laser ablation of gold in a liquid that contains the nucleic acid sequence.

5. Method according to claim 4, **characterized in that** the ultrashort laser pulse has a pulse duration of 10 fs to 15 ps at a wavelength of at least 330 nm up to maximally 1030 nm.

6. Method according to one of claims 2 to 4, **characterized in that** the ablation time is 10 to 200 s at a pulse energy of 50 to 200 µJ and a pulse duration of 100 to 140 fs at 800 nm.

7. Method according to one of claims 2 to 6, **characterized in that** the nucleic acid sequence contains at least one reactive group selected from the group containing keto groups, amine groups, thiol groups, and carboxyl groups.

8. Method according to one of the preceding claims, **characterized in that** the oligonucleotide is PNA.

9. Method according to one of the preceding claims, **characterized in that** the nucleic acid sequence has the base sequence 5' agc aca tct cgg tcc ctg 3' (SEQ ID NO. 1) or a base sequence complementary thereto.

10. Method according to one of the preceding claims, **characterized in that** spermatozoa are deflected into one of at least two fractions in dependence on the measured signal.

11. Method according to one of claims 1 to 8, **characterized in that** gametes are heat-treated by targeted laser radiation in dependence on the detected signal.

12. Method according to one of the preceding claims, **characterized in that** the fraction of nonhuman mammalian gametes is intact and viable.

13. Method according to one of the preceding claims, **characterized in that** the mammalian gametes contain fluoride and/or antioxidation agent.

14. Method according to one of the preceding claims, **characterized in that** the gametes are spermatozoa.

15. Fraction of nonhuman mammalian gametes having a predominant portion of fertile gametes containing an X-chromosome or a Y-chromosome, obtainable by a method according to one of the preceding claims, and containing a detection conjugate that has an oligonucleotide having a sex-chromosome-specific nucleic acid sequence having a bound fluorochrome and having a quencher bound at a distance from the fluorochrome.

16. Fraction of nonhuman mammalian gametes having a predominant portion of fertile gametes containing an X-chromosome or a Y-chromosome, obtainable by a method according to one of claims 1 to 14, and containing a detection conjugate that has an oligonucleotide having a sex-chromosome-specific nucleic acid sequence that is bound to a gold nanoparticle.

17. Fraction according to one of claims 15 or 16, **characterized in that** the gametes are spermatozoa.

## Revendications

1. Procédé de fabrication d'une fraction de gamètes de mammifères non humains présentant une portion prédominante de gamètes fertilisables contenant des chromosomes X ou contenant des chromosomes Y par détection du rayonnement émis par un conjugué de détection par irradiation de rayonnement avec une longueur d'ondes d'excitation, **caractérisé en ce que** le conjugué de détection présente un oligonucléotide possédant une séquence d'acide nucléique spécifique au chromosome sexuel avec un fluorochrome lié de même qu'un quencher lié à une distance du fluorochrome.

2. Procédé de fabrication d'une fraction de gamètes de mammifères non humains présentant une portion prédominante de gamètes fertilisables contenant des chromosomes X ou contenant des chromosomes Y par détection du rayonnement émis par conjugué de détection par irradiation de rayonnement avec une longueur d'ondes d'excitation, **caractérisé en ce que** le conjugué de détection présente un oligonucléotide possédant une séquence d'acide nucléique spécifique au chromosome sexuel, séquence liée immédiatement à une nanoparticule d'or.

3. Procédé selon la revendication 2, **caractérisé en ce que** la nanoparticule d'or présente une taille de 1 à 100 nm.

4. Procédé selon la revendication 2 ou 3, **caractérisé en ce que** l'on peut obtenir le conjugué de détection par production de nanoparticules d'or par érosion par impulsions laser ultracourtes d'or dans un liquide contenant la séquence d'acide nucléique.

5. Procédé selon la revendication 4, **caractérisé en ce que** l'impulsion laser ultracourte présente une durée d'impulsion de 10 fs à 15 ps à une longueur d'ondes d'au moins 330 nm jusqu'à 1030 nm au maximum.

6. Procédé selon l'une quelconque des revendications 2 à 4, **caractérisé en ce que** le temps d'érosion va de 10 de 200 s à une énergie d'impulsion de 50 à 200 µJ et une durée d'impulsion de 100 à 140 fs à 800 nm.

7. Procédé selon l'une quelconque des revendications 2 à 6, **caractérisé en ce que** la séquence d'acide nucléique contient au moins un groupe réactif que l'on choisit parmi le groupe comprenant les groupes cétones, les groupes aminés, les groupes thiols et les groupes carboxyliques.

8. Procédé selon l'une des revendications précédentes, **caractérisé en ce que** l'oligonucléotide est un APN.

9. Procédé selon l'une des revendications précédentes, **caractérisé en ce que** la séquence d'acide nucléique présente la suite de base 5' agc aca tct egg tec ctg 3' (Seq.-ID No. 1) ou une suite de base complémentaire de celle-ci.

10. Procédé selon l'une des revendications précédentes, **caractérisé en ce que** spermatozoïdes sont dirigés vers une d'au moins deux fractions en fonction du signal mesuré.

11. Procédé selon l'une quelconque des revendications 1 à 8, **caractérisé en ce que** les gamètes subissent un traitement thermique en fonction du signal détecté par un rayonnement laser ciblé.

12. Procédé selon l'une des revendications précédentes, **caractérisé en ce que** la fraction de gamètes de mammifères non humains est intacte et viable.

13. Procédé selon l'une des revendications précédentes, **caractérisé en ce que** les gamètes de mammifères présentent une teneur en fluorure et/ou agents antioxidants.

14. Procédé selon l'une des revendications précédentes, **caractérisé en ce que** les gamètes sont des spermatozoïdes.

15. Fraction de gamètes de mammifères non humains présentant une portion prédominante de gamètes fertilisables contenant des chromosomes X ou contenant des chromosomes Y, que l'on obtient par un procédé selon l'une des revendications précédentes, et une teneur en un conjugué de détection présentant un oligonucléotide possédant une séquence d'acide nucléique spécifique au chromosome sexuel avec un fluorochrome lié de même qu'un quencher lié à une certaine distance du fluorochrome.

16. Fraction de gamètes de mammifères non humains présentant une portion prédominante de gamètes fertilisables contenant des chromosomes X ou contenant des chromosomes Y, que l'on obtient par un procédé selon l'une des revendications 1 à 14, et d'une teneur en conjugué de détection, comportant un oligonucléotide possédant une séquence d'acide nucléique spécifique au chromosome sexuel, séquence liée immédiatement à une nanoparticule d'or.

17. Fraction selon l'une quelconque des revendications 15 ou 16, **caractérisée en ce que** les gamètes sont des spermatozoïdes.


### SEQUENCE LISTING

<110> Masterrind GmbH
<120> verfahren zur zellidentifikation und zellsortierung
<130> M1010PCT
<160> 4
<170> PatentIn version 3.5
<210> 1
   <211> 18
   <212> DNA
   <213> Artificial sequence
<220>
   <223> seq.-ID Nr. 1: Y-chromosom-spezifisches Oligonukleotid
<400> 1
   agcacatctc ggtccctg 18
<210> 2
   <211> 18
   <212> DNA
   <213> Artificial Sequence
<220>
   <223> seq.-ID Nr. 2: Y-Chromosom-spezifisches Oligonukleotid
<400> 2
   ggcgactgtg caagcaga 18
<210> 3
   <211> 17
   <212> DNA
   <213> Artificial Sequence
<220>
   <223> Seq.-ID Nr. 3: Y-chromosom-spezifisches Oligonukleotid
<400> 3
   agagactgtg gaaccgg 17
<210> 4
   <211> 17
   <212> DNA
   <213> Artificial Sequence
<220>
   <223> Seq.-ID Nr. 4: Y-chromosom-spezifisches Oligonukleotid
<400> 4
   ggcgattgtt caaccag 17